# EUROPEAN PATENT APPLICATION

(11) **EP 4 846 994 A1**
(43) Date of publication of application: **02.12.2026**
(21) Application number: 24927209.7
(22) Date of filing: 29.02.2024
(51) Int. Cl.: A61F 13/53, A61F 13/505, A61F 13/511

(54) **ABSORBENT ARTICLE AND ABSORBENT ARTICLE SET**

(71) Applicant: Unicharm Corporation, Shikokuchuo-city, Ehime 799-0111 (JP)
(72) Inventor: ISHII, Yudai, Kanonji-shi, Kagawa 769-1602 (JP); TANAKA, Suguru, Kanonji-shi, Kagawa 769-1602 (JP); SHIMADA, Yusei, Kanonji-shi, Kagawa 769-1602 (JP); SHIMAZU, Takeshi, Kanonji-shi, Kagawa 769-1602 (JP)
(74) Representative: Dolleymores
(86) International application number: PCT/JP2024/007637
(87) International publication number: WO 2025/182043

(57) **Abstract**

The absorbent article 1 includes a top sheet 21, a back sheet 22, and an absorbent body 23 including an absorbent core 31. The absorbent body 23 has a pair of colored regions A1 disposed on both sides of a center line CL1 so as to overlap with the absorbent core 31 in the thickness direction Z, and a non-colored region A2 disposed between the pair of colored regions A1 in the width direction X on the same side as the side on which the pair of colored regions A1 is provided with respect to the absorbent core 31. An outer edge portion of each of the pair of colored regions A1 in the width direction X is disposed so as to overlap with an edge 31c of the absorbent core 31 in the width direction X. The quantity of sheet materials laminated on the pair of colored regions A1 is equal to or less than the quantity of sheet materials laminated on the non-colored region A2.

## Description

### Technical Field

The present disclosure relates to an absorbent article and an absorbent article set.

### Background Art

Conventionally, an absorbent article is known in which a pair of side absorbent bodies is provided on both sides of a central absorbent body, and the pair of side absorbent bodies is configured to easily stand up, thereby preventing leakage of excreta (refer to Patent Literature 1).

### Citation List

### Patent Literature

[Patent Literature 1] Japanese Unexamined Patent Publication No. 2013-102888

### Summary of Invention

### Technical Problem

The color of an absorbent body including an absorbent core is generally white, and is often of a similar color to other constituent materials included in the absorbent article. For this reason, even if the leakage prevention effect is improved by increasing the width of the absorbent core as in the absorbent article described in Patent Literature 1, the user of the absorbent article cannot easily visually recognize that the absorbent core has become wider. As a result, it has been difficult for the user to feel a sense of security with respect to the absorbent article (in other words, a sense of expectation that leakage of excreta will be effectively prevented by the absorbent core, and a sense of actual effect that leakage of excreta around the legs is being effectively suppressed by the absorbent article). Fostering such a sense of security with respect to the absorbent article is extremely important, particularly in cases where the absorbent article is used for a long time or used at night, in order to eliminate the user's anxiety and allow the user to lead a comfortable life.

Therefore, an object of one aspect of the present disclosure is to provide an absorbent article and an absorbent article set capable of effectively improving the user's sense of security.

### Solution to Problem

An absorbent article according to one aspect of the present disclosure is an absorbent article in which a ventral region positioned on the ventral side of a wearer in a worn state, a dorsal region positioned on the dorsal side of the wearer in the worn state, and a crotch region positioned between the ventral region and the dorsal region are provided along a longitudinal direction, the absorbent article including: a liquid-permeable top sheet positioned on the skin-facing side of the wearer in the worn state; a liquid-impermeable back sheet positioned on the non-skin-facing side of the wearer in the worn state; and an absorbent body disposed between the top sheet and the back sheet and including an absorbent core that absorbs excreta of the wearer, wherein the absorbent body has a pair of colored regions disposed on both sides of a center line parallel to the longitudinal direction and passing through the center of the absorbent body in a width direction perpendicular to the longitudinal direction so as to overlap with the absorbent core in a thickness direction perpendicular to the longitudinal direction and the width direction, and a non-colored region disposed between the pair of colored regions in the width direction on the same side as the side on which the pair of colored regions is provided with respect to the absorbent core, wherein an outer edge portion of each of the pair of colored regions in the width direction is disposed so as to overlap with an edge of the absorbent core in the width direction, and a quantity of sheet materials laminated on the pair of colored regions on a side opposite to the side on which the absorbent core is disposed with respect to the pair of colored regions is equal to or less than a quantity of sheet materials laminated on the non-colored region on a side opposite to the side on which the absorbent core is disposed with respect to the non-colored region.

In the absorbent article of the above aspect, the pair of colored regions having outer edge portions overlapping with the edges of the absorbent core in the width direction is provided. The quantity (number or amount) of sheet materials laminated on the pair of colored regions is equal to or less than the quantity of sheet materials laminated on the non-colored region. That is, the absorbent article is configured such that the visibility of the pair of colored regions is not impaired more than necessary by not disposing a greater quantity of sheet materials on the pair of colored regions than on the non-colored region. This allows the user to appropriately visually recognize the pair of colored regions, and allows the user to easily grasp the position of the edges of the absorbent core in the width direction. As a result, it is possible to give the user a sense of expectation that leakage of excreta will be effectively prevented by the absorbent core disposed up to the positions overlapping with the outer edge portions of the pair of colored regions. Furthermore, after use of the absorbent article or the like, when it is confirmed that lateral leakage of excreta has not occurred, a sense of security with respect to the absorbent article (in other words, a sense of trust in the absorbent article based on the actual feeling that the effect that was expected in advance as described above was actually obtained) can be suitably fostered. Therefore, according to the above absorbent article, the user's sense of security can be effectively improved.

An absorbent article set according to another aspect of the present disclosure is an absorbent article set including the above absorbent article and an absorbent pad used by being attached to the absorbent article, wherein the absorbent article is a pants-type or tape-type disposable diaper, the pair of colored regions and the non-colored region are disposed on the skin-facing side of the wearer relative to the absorbent core, and in a state where the absorbent pad is attached to the skin-facing side of the absorbent article such that the center line passes through the center of the absorbent pad in the width direction, at the central position of the absorbent body in the longitudinal direction, the outer edge portions of the pair of colored regions in the width direction are positioned further outward than the outer end portions of the absorbent pad in the width direction.

In the absorbent article set of the above aspect, even in a state where the absorbent pad is attached to the absorbent article for use in combination with the absorbent article, the user can visually recognize the outer edge portions of the pair of colored regions at the central position of the absorbent body in the longitudinal direction. This allows the user to visually grasp that the absorbent article has an absorbent core that is even wider than the absorbent pad in the region where lateral leakage is of concern (that is, the central position of the absorbent body in the longitudinal direction). As a result, the user's sense of security can be effectively improved.

### Advantageous Effects of Invention

According to the present disclosure, it is possible to provide an absorbent article and an absorbent article set capable of effectively improving the user's sense of security.

### Brief Description of Drawings

[FIG. 1] is a perspective view schematically showing an absorbent article according to an embodiment.
[FIG. 2] is a plan view of the absorbent article as viewed from the non-skin-facing side.
[FIG. 3] is a plan view of the absorbent article as viewed from the skin-facing side.
[FIG. 4] is a schematic view of a cross-sectional structure of the absorbent article taken along line IV-IV in FIG. 3.
[FIG. 5] is a schematic view of a cross-sectional structure of the absorbent article taken along line V-V in FIG. 3. (A) is a view schematically showing a state in which leakage-proof gathers are standing upright. (B) is a view schematically showing a state in which the leakage-proof gathers are laid down to the maximum toward the inner side.
[FIG. 6] is a view showing an arrangement example of recessed portions of the absorbent core.
[FIG. 7] is a view showing a modification example of the width of the colored region.
[FIG. 8] is a view showing a modification example of the absorbent body.
[FIG. 9] is a view showing examples of coloring patterns of the colored region.
[FIG. 10] is a plan view of an absorbent article set according to an embodiment as viewed from the skin-facing side.

### Description of Embodiments

At least the following aspects [1] to [19] will become apparent from the description of the present specification and the accompanying drawings.
[1] An absorbent article in which a ventral region positioned on a ventral side of a wearer in a worn state, a dorsal region positioned on a dorsal side of the wearer in the worn state, and a crotch region positioned between the ventral region and the dorsal region are provided along a longitudinal direction, the absorbent article comprising:
   a liquid-permeable top sheet positioned on a skin-facing side of the wearer in the worn state;
   a liquid-impermeable back sheet positioned on a non-skin-facing side of the wearer in the worn state;
   an absorbent body disposed between the top sheet and the back sheet and including an absorbent core that absorbs excreta of the wearer,
   wherein the absorbent body has:
      a pair of colored regions disposed on both sides of a center line parallel to the longitudinal direction and passing through a center of the absorbent body in a width direction perpendicular to the longitudinal direction so as to overlap with the absorbent core in a thickness direction perpendicular to the longitudinal direction and the width direction; and
      a non-colored region disposed between the pair of colored regions in the width direction on the same side as the side on which the pair of colored regions is provided with respect to the absorbent core,
      wherein an outer edge portion of each of the pair of colored regions in the width direction is disposed so as to overlap with an edge of the absorbent core in the width direction, and
      a quantity of sheet materials laminated on the pair of colored regions on a side opposite to the side on which the absorbent core is disposed with respect to the pair of colored regions is equal to or less than a quantity of sheet materials laminated on the non-colored region on a side opposite to the side on which the absorbent core is disposed with respect to the non-colored region.
[2] The absorbent article according to [1], wherein the pair of colored regions and the non-colored region are disposed on the skin-facing side of the wearer relative to the absorbent core.
[3] The absorbent article according to [1], wherein the pair of colored regions and the non-colored region are disposed on the non-skin-facing side of the wearer relative to the absorbent core.
[4] The absorbent article according to any one of [1] to [3], further comprising a pair of leakage-proof gathers provided on both sides of the absorbent body in the width direction, both end portions of the leakage-proof gathers in the longitudinal direction being fixed and a portion between both end portions being configured to be able to stand upright by contracting in the longitudinal direction,
   wherein in a state where the leakage-proof gathers are folded toward an inner side in the width direction, an inner edge portion of each of the pair of colored regions in the width direction is positioned further inward in the width direction than top portions of the leakage-proof gathers.
[5] The absorbent article according to any one of [1] to [4], further comprising a pair of leakage-proof gathers provided on both sides of the absorbent body in the width direction, both end portions of the leakage-proof gathers in the longitudinal direction being fixed and a portion between both end portions being configured to be able to stand upright by contracting in the longitudinal direction,
   wherein in a state where the leakage-proof gathers are folded toward an inner side in the width direction, at a central position of the absorbent body in the longitudinal direction, edges of the absorbent core in the width direction are positioned further outward in the width direction than top portions of the leakage-proof gathers.
[6] The absorbent article according to any one of [1] to [5], wherein the absorbent core is formed with a substantially uniform width over an entire region in the longitudinal direction.
[7] The absorbent article according to any one of [1] to [6], wherein a portion of the absorbent core included in the crotch region is provided with a pair of recessed portions on both sides of the center line, each of the recessed portions extending in the longitudinal direction and having a thickness of the absorbent core in the thickness direction smaller than that of a surrounding area.
[8] The absorbent article according to [7], wherein each of the pair of recessed portions has a ventral curved portion that curves toward an outer side in the width direction as it extends from the central position of the recessed portion in the longitudinal direction toward the ventral side, and a dorsal curved portion that curves toward the outer side in the width direction as it extends from the central position of the recessed portion toward the dorsal side.
[9] The absorbent article according to [7] or [8], wherein at least a part of each of the pair of recessed portions overlaps with a corresponding one of the pair of colored regions in the thickness direction.
[10] The absorbent article according to any one of [7] to [9], wherein each of the pair of recessed portions overlaps with a corresponding one of the pair of colored regions in the thickness direction over more than half of its area.
[11] The absorbent article according to any one of [7] to [10], wherein an entirety of each of the pair of recessed portions overlaps with a corresponding one of the pair of colored regions in the thickness direction.
[12] The absorbent article according to any one of [1] to [11], wherein at a central position of the absorbent body in the longitudinal direction, a sum of widths of the pair of colored regions in the width direction is equal to or greater than a width of the non-colored region in the width direction.
[13] The absorbent article according to any one of [1] to [11], wherein at a central position of the absorbent body in the longitudinal direction, a sum of widths of the pair of colored regions in the width direction is equal to or less than a width of the non-colored region in the width direction.
[14] The absorbent article according to any one of [1] to [13], wherein each of the pair of colored regions is disposed over at least an entire region of the crotch region in the longitudinal direction.
[15] The absorbent article according to any one of [1] to [14], wherein each of the pair of colored regions is disposed so as to overlap with regions on both sides of a central region in the longitudinal direction when the absorbent article is divided into three equal parts in the longitudinal direction.
[16] The absorbent article according to any one of [1] to [15], wherein edges of each of the pair of colored regions in the longitudinal direction overlap with edges of the absorbent core in the longitudinal direction.
[17] The absorbent article according to any one of [1] to [16], wherein a portion of the absorbent core included in the crotch region is provided with a groove portion extending in the longitudinal direction along the center line and having a thickness of the absorbent core in the thickness direction smaller than that of a surrounding area.
[18] The absorbent article according to [17], further comprising a stretchable member for stretching and contracting, in the longitudinal direction, at least a part of the portion of the absorbent core included in the crotch region and extending along the center line.
[19] An absorbent article set including an absorbent article having at least the configuration of [2] above and an absorbent pad used by being attached to the absorbent article,
   wherein the absorbent article is a pants-type or tape-type disposable diaper, and
   in a state where the absorbent pad is attached to the skin-facing side of the inner body of the absorbent article such that the center line passes through the center of the absorbent pad in the width direction, at a central position of the absorbent body in the longitudinal direction, outer edge portions of the pair of colored regions in the width direction are positioned further outward than outer end portions of the absorbent pad in the width direction.

### [Embodiment]

Hereinafter, embodiments of the present disclosure will be described in detail with reference to the drawings. In the following description, the same or equivalent elements will be given the same reference numerals, and redundant description thereof will be omitted. The drawings may be partially simplified or exaggerated for ease of understanding, and dimensional ratios are not limited to those shown in the drawings.

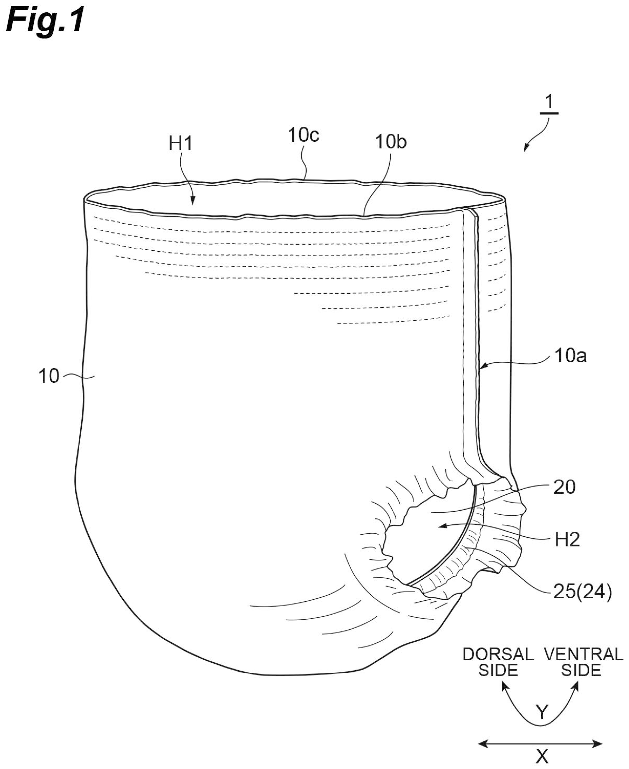

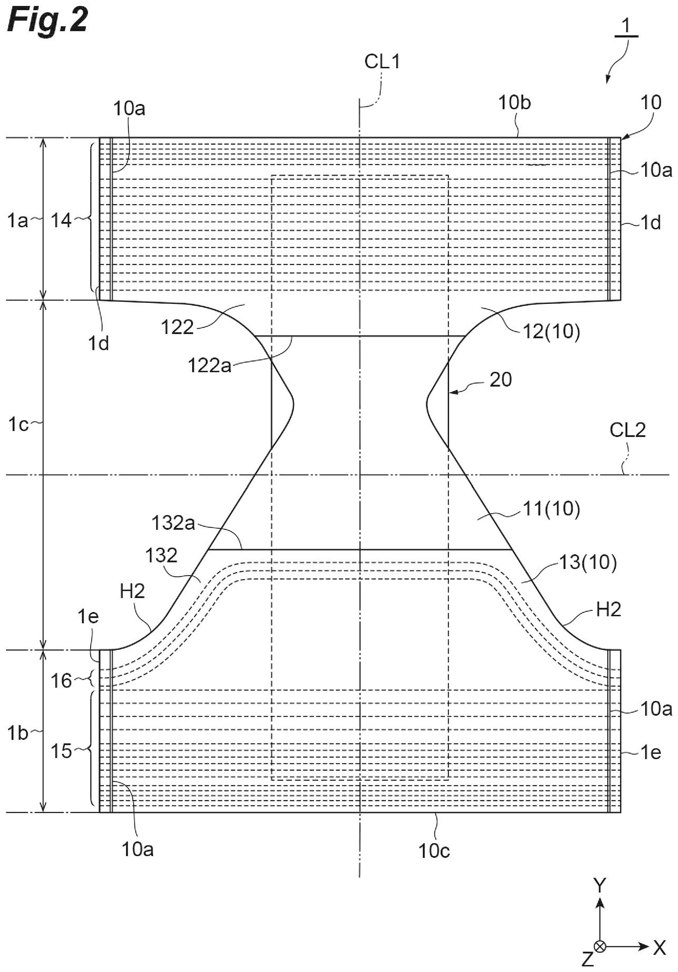

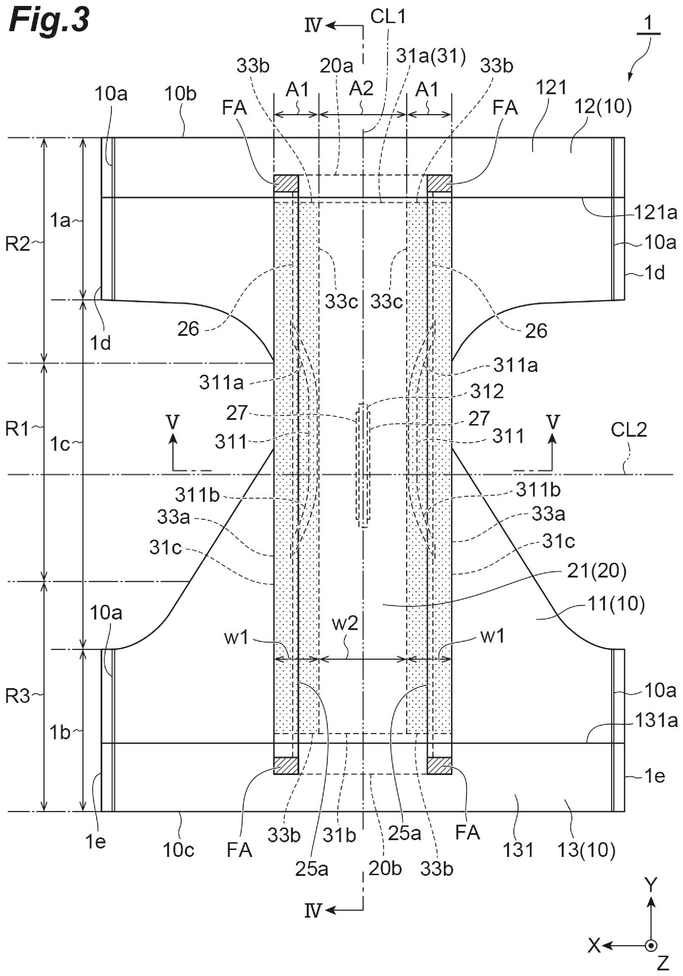

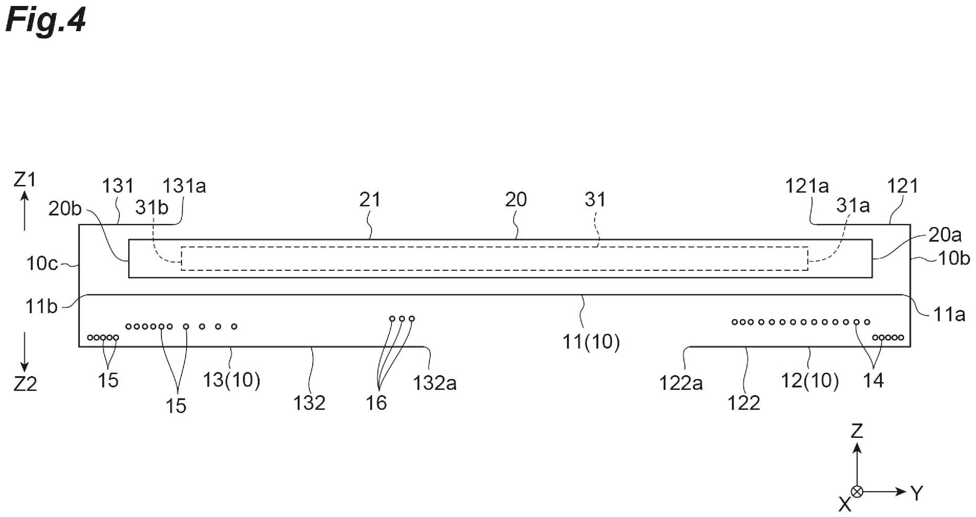

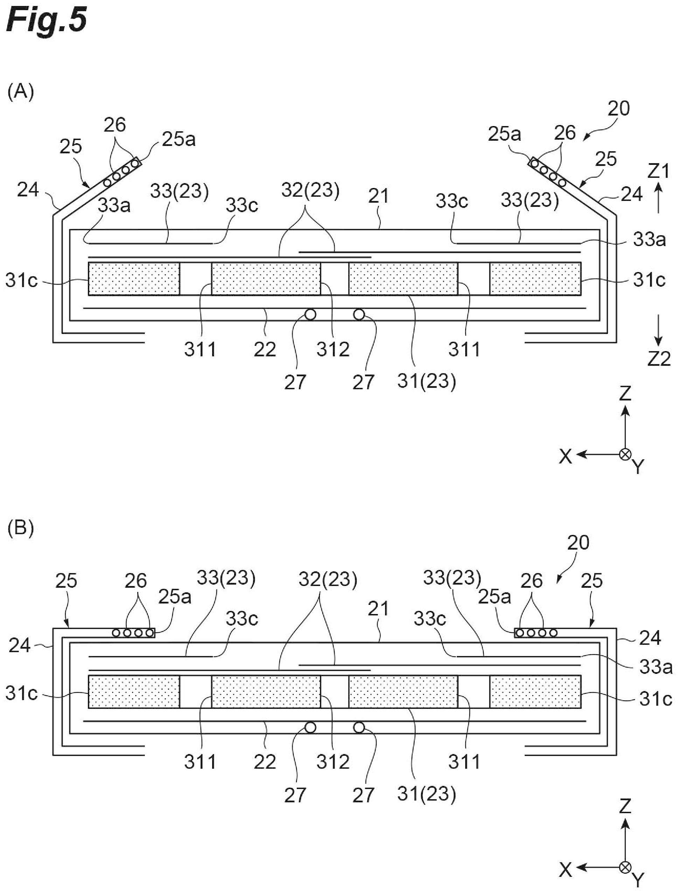

Hereinafter, an absorbent article 1 according to an embodiment will be described. FIG. 1 is a perspective view of the absorbent article 1. FIG. 2 is a plan view of the absorbent article 1 as viewed from the non-skin-facing side Z2, and FIG. 3 is a plan view of the absorbent article 1 as viewed from the skin-facing side Z1. FIG. 4 is a schematic view of a cross-sectional structure taken along line IV-IV in FIG. 3. (A) and (B) of FIG. 5 are schematic views of a cross-sectional structure taken along line V-V in FIG. 3. (A) of FIG. 5 schematically shows a state in which leakage-proof gathers 25 to be described later are standing upright, and (B) of FIG. 5 schematically shows a state in which the leakage-proof gathers 25 are laid down to the maximum toward the inner side.

In the following description, the "wearer" is a person who wears the absorbent article 1. The "skin-facing side" is the side facing the skin of the wearer in a state where the absorbent article 1 is worn by the wearer (hereinafter referred to as the "worn state"). The "non-skin-facing side" is the side directed away from the skin of the wearer in the worn state. The "user" of the absorbent article 1 includes the above-described wearer and also includes an assistant (caregiver/care provider) who raises or cares for a wearer who is a child or a person requiring care.

As shown in FIG. 1, as an example, the absorbent article 1 is a pants-type disposable diaper that absorbs excreta such as urine of the wearer. That is, the absorbent article 1 has a waist opening H1 through which the torso of the wearer is inserted, and a pair of leg openings H2 through which both legs of the wearer are inserted (in FIG. 1, the leg opening H2 through which the left leg is inserted is shown). The absorbent article 1 is formed of an outer body 10 facing the outer side (non-skin-facing side Z2) in the worn state and an inner body 20 fixed to the inner side of the outer body 10. The absorbent article 1 is configured to be cuttable by tearing a pair of side seal portions 10a of the outer body 10 provided on both the left and right sides of the wearer.

FIGS. 2 and 3 show the absorbent article 1 in an unfolded state in which the outer body 10 is cut along the pair of side seal portions 10a and the absorbent article 1 is extended to a state where no wrinkles are formed. In the following description, unless otherwise specified, the shape and positional relationship of each part of the absorbent article 1 in the unfolded state will be described.

As shown in FIGS. 2 and 3, the absorbent article 1 has a configuration in which a ventral region 1a positioned on the ventral side of the wearer in the worn state, a dorsal region 1b positioned on the dorsal side of the wearer in the worn state, and a crotch region 1c positioned between the ventral region 1a and the dorsal region 1b are provided along a longitudinal direction Y.

The absorbent article 1 extends in the width direction X and the longitudinal direction Y in the unfolded state. The longitudinal direction Y is the direction in which the ventral region 1a, the crotch region 1c, and the dorsal region 1b are arranged. In the unfolded state, the longitudinal direction Y is the direction in which the ventral region 1a and the dorsal region 1b face each other with the crotch region 1c interposed therebetween. The width direction X is a direction perpendicular to the longitudinal direction Y and corresponds to the left-right direction of the wearer in the worn state. The thickness direction Z is a direction perpendicular to both the width direction X and the longitudinal direction Y, and is the direction in which the outer body 10 and the inner body 20 overlap each other. In the following description, the side on which the ventral region 1a is positioned in the longitudinal direction Y of the absorbent article 1 may be simply referred to as the "ventral side", and the side on which the dorsal region 1b is positioned in the longitudinal direction Y may be simply referred to as the "dorsal side".

When the absorbent article 1 is a pants-type diaper as in the present embodiment, the ventral region 1a is defined as the portion of the absorbent article 1 included in the range from the ventral end portion 10b of the outer body 10 to the dorsal end portions of the ventral side seal portions 10a in the unfolded state (refer to FIGS. 2 and 3). Similarly, the dorsal region 1b is defined as the portion of the absorbent article 1 included in the range from the dorsal end portion 10c of the outer body 10 to the ventral end portions of the dorsal side seal portions 10a in the unfolded state. Note that when the absorbent article 1 has a configuration other than a pants-type diaper (for example, a tape-type diaper or an absorbent pad), the ventral region 1a and the dorsal region 1b may be defined by other methods. For example, when the entire absorbent article 1 is divided into four equal parts in the longitudinal direction Y, the two central regions may be defined as the crotch region 1c, and the regions on both sides thereof may be defined as the ventral region 1a and the dorsal region 1b.

### (Configuration of Outer Body)

As shown in FIGS. 2 to 4, the outer body 10 has a main body sheet 11, a ventral sheet 12, and a dorsal sheet 13. As an example, the main body sheet 11 is a sheet member that defines the overall outer shape of the outer body 10 as viewed from the thickness direction Z (in the present embodiment, an hourglass shape as an example). However, the configuration of the main body sheet 11 is not limited to the above. For example, the main body sheet 11 may have a so-called three-piece configuration formed of two sheet members corresponding to the ventral side and the dorsal side, respectively, and a sheet member disposed on the non-skin-facing side Z2 of the inner body 20. The ventral sheet 12 is disposed so as to overlap with the main body sheet 11 in the ventral region 1a. The dorsal sheet 13 is disposed so as to overlap with the main body sheet 11 in the dorsal region 1b. The main body sheet 11 is formed of, for example, an SMS nonwoven fabric or the like. The ventral sheet 12 and the dorsal sheet 13 are formed of, for example, a spunbond nonwoven fabric or the like.

The ventral sheet 12 is folded back so as to sandwich the ventral end portion 11a of the main body sheet 11. That is, the ventral sheet 12 has an inner portion 121 positioned on the inner side (skin-facing side Z1) relative to the main body sheet 11, and an outer portion 122 positioned on the non-skin-facing side Z2 (outer side) relative to the main body sheet 11.

An end portion 121a of the folded-back inner portion 121 is positioned on the dorsal side relative to the ventral end portion 20a of the inner body 20 and on the ventral side relative to the ventral end portion 31a of the absorbent core 31 included in the inner body 20.

An end portion 122a of the folded-back outer portion 122 is positioned further toward the dorsal side than the end portion 121a of the inner portion 121. A plurality (19 in the present embodiment) of waist stretchable members 14 are arranged along the longitudinal direction Y between the outer portion 122 and the main body sheet 11 in the ventral region 1a. As shown in FIG. 2, each waist stretchable member 14 extends parallel to the width direction X between the edges 1d of the ventral region 1a of the absorbent article 1 in the width direction X. Therefore, the ventral waist portion of the outer body 10 stretches and contracts in the width direction X in a state of fitting the waist of the wearer. The plurality of waist stretchable members 14 are formed of an elastic material such as rubber thread or spandex.

The dorsal sheet 13 is folded back so as to sandwich the dorsal end portion 11b of the main body sheet 11. That is, the dorsal sheet 13 has an inner portion 131 positioned on the inner side (skin-facing side Z1) relative to the main body sheet 11, and an outer portion 132 positioned on the non-skin-facing side Z2 (outer side) relative to the main body sheet 11.

An end portion 131a of the folded-back inner portion 131 is positioned on the ventral side relative to the dorsal end portion 20b of the inner body 20 and on the dorsal side relative to the dorsal end portion 31b of the absorbent core 31 included in the inner body 20.

An end portion 132a of the folded-back outer portion 132 is positioned further toward the ventral side than the end portion 131a of the inner portion 131. A plurality (15 in the present embodiment) of waist stretchable members 15 are arranged along the longitudinal direction Y between the outer portion 132 and the main body sheet 11 in the dorsal region 1b. As shown in FIG. 2, each waist stretchable member 15 extends parallel to the width direction X between the edges 1e of the dorsal region 1b of the absorbent article 1 in the width direction X. Therefore, the dorsal waist portion of the outer body 10 also stretches and contracts in the width direction X in a state of fitting the waist of the wearer, similarly to the ventral waist portion provided with the above-described waist stretchable members 14. The plurality of waist stretchable members 15 are formed of an elastic material such as rubber thread or spandex.

Between the outer portion 132 and the main body sheet 11, a plurality (3 in the present embodiment) of leg stretchable members 16 are arranged along the longitudinal direction Y on the ventral side relative to the plurality of waist stretchable members 15. As shown in FIG. 2, each leg stretchable member 16 extends in the width direction X between the edges 1e of the dorsal region 1b of the absorbent article 1, and includes a portion extending in a curved shape along the edge of the dorsal portion of each of the pair of leg openings H2. The outer body 10 stretches and contracts so as to fit the legs of the wearer by the plurality of leg stretchable members 16. The plurality of leg stretchable members 16 are formed of an elastic material such as rubber thread or spandex.

The inner portion 121 of the ventral sheet 12 and the inner body 20 are bonded to each other by a hot melt adhesive (not shown) disposed between the inner portion 121 and the inner body 20. In addition, the outer portion 122 of the ventral sheet 12 and the main body sheet 11 are bonded to each other by a hot melt adhesive (not shown) disposed between the outer portion 122 and the main body sheet 11 so as to sandwich the plurality of waist stretchable members 14. Similarly, the inner portion 131 of the dorsal sheet 13 and the inner body 20 are bonded to each other by a hot melt adhesive (not shown) disposed between the inner portion 131 and the inner body 20. In addition, the outer portion 132 of the dorsal sheet 13 and the main body sheet 11 are bonded to each other by a hot melt adhesive (not shown) disposed between the outer portion 132 and the main body sheet 11 so as to sandwich the plurality of waist stretchable members 15 and the plurality of leg stretchable members 16. In addition, the main body sheet 11 and the inner body 20 are bonded to each other by a hot melt adhesive (not shown) disposed between the main body sheet 11 and the inner body 20 (back sheet 22). By joining the respective members to each other as described above, the absorbent article 1 has a configuration in which the outer body 10 and the inner body 20 are integrally joined.

### (Configuration of Inner Body)

As shown in FIGS. 2, 3, and 5, the inner body 20 has a rectangular shape extending in the longitudinal direction Y and the width direction X as viewed from the thickness direction Z, with the length in the longitudinal direction Y being longer than the length in the width direction X. The inner body 20 has a top sheet 21, a back sheet 22, an absorbent body 23, and a pair of side sheets 24.

The top sheet 21 is positioned on the skin-facing side Z1 of the wearer in the worn state and forms the surface that comes into contact with the skin of the wearer. The top sheet 21 is formed of a nonwoven fabric having liquid permeability (water permeability) such as a spunbond nonwoven fabric, and covers the absorbent body 23 from the skin-facing side Z1. Typically, the top sheet 21 is white and plain. The top sheet 21 is bonded to the surface of the absorbent body 23 on the skin-facing side Z1 (the surfaces of the white tissues 32 and the colored tissues 33 to be described later) by, for example, a hot melt adhesive (not shown).

The back sheet 22 is a sheet positioned on the non-skin-facing side Z2 of the wearer in the worn state. Specifically, the back sheet 22 is positioned on the opposite side from the top sheet 21 with the absorbent body 23 interposed therebetween. The back sheet 22 is formed of, for example, a film having liquid impermeability (water impermeability), and covers the absorbent body 23 from the non-skin-facing side Z2. The back sheet 22 is bonded to the surface of the absorbent body 23 on the non-skin-facing side Z2 by, for example, a hot melt adhesive (not shown).

The absorbent body 23 is disposed between the top sheet 21 and the back sheet 22 of the inner body 20. As shown in FIG. 5, the absorbent body 23 includes an absorbent core 31 that absorbs excreta such as urine of the wearer that has passed through the top sheet 21, a pair of white tissues 32 that cover the skin-facing side Z1 of the absorbent core 31, and a pair of colored tissues 33 disposed on the pair of white tissues 32. The width (length in the width direction X) of the absorbent body 23 (absorbent core 31) substantially coincides with the width of the inner body 20. The length (length in the longitudinal direction Y) of the absorbent body 23 (absorbent core 31) is slightly shorter than the length of the inner body 20. Specifically, the ventral end portion 31a of the absorbent core 31 is positioned on the dorsal side relative to the ventral end portion 20a of the inner body 20 (top sheet 21 and the like). In addition, the dorsal end portion 31b of the absorbent core 31 is positioned on the ventral side relative to the dorsal end portion 20b of the inner body 20 (top sheet 21 and the like).

The absorbent core 31 is laminated on the back sheet 22. The absorbent core 31 includes, for example, pulp fibers and a super absorbent polymer (SAP), and absorbs urine of the wearer. As the pulp of the absorbent core 31, for example, cellulose fibers such as chemical pulp, cellulose fibers, rayon, and acetate are used. As the super absorbent polymer, for example, a starch-based, acrylic acid-based, or amino acid-based particulate or fibrous polymer is used.

The pair of white tissues 32 is disposed so as to cover the skin-facing side Z1 of the absorbent core 31. As an example, each of the pair of white tissues 32 extends from the outer edge portion of the absorbent core 31 in the width direction X to the central portion in the width direction X, and is disposed so as to overlap with the other at the central portion in the width direction X. Each of the pair of white tissues 32 is formed in a rectangular shape extending from the ventral end portion 31a to the dorsal end portion 31b of the absorbent core 31 when viewed from the thickness direction Z. That is, the edges of each white tissue 32 in the longitudinal direction Y overlap with the edges of the absorbent core 31 in the longitudinal direction Y (the ventral end portion 31a and the dorsal end portion 31b).

The pair of colored tissues 33 are tissues colored in a color other than white (blue as an example). The pair of colored tissues 33 is disposed on both sides of a center line CL1 passing through the center of the absorbent body 23 (absorbent core 31) in the width direction X and parallel to the longitudinal direction Y so as to overlap with the absorbent core 31 in the thickness direction Z. The outer end portion 33a of each of the pair of colored tissues 33 in the width direction X is disposed so as to overlap with the edge 31c of the absorbent core 31 in the width direction X. Each of the pair of colored tissues 33 is formed in a rectangular shape extending from the ventral end portion 31a to the dorsal end portion 31b of the absorbent core 31 when viewed from the thickness direction Z. That is, the edges 33b (refer to FIG. 3) of each colored tissue 33 in the longitudinal direction Y overlap with the edges of the absorbent core 31 in the longitudinal direction Y (the ventral end portion 31a and the dorsal end portion 31b).

Here, since the top sheet 21 is a liquid-permeable nonwoven fabric as described above, the color of the member on the non-skin-facing side Z2 of the top sheet 21 is visible through the top sheet 21 from the skin-facing side Z1 of the top sheet 21. For this reason, it is possible to visually recognize the color of each of the pair of colored tissues 33 from the skin-facing side Z1. That is, as shown in FIG. 3, a pair of colored regions A1 is formed by the pair of colored tissues 33. In addition, a non-colored region A2 is formed on the same side as the side on which the pair of colored regions A1 is provided with respect to the absorbent core 31 (the skin-facing side Z1 in the present embodiment), disposed between the pair of colored regions A1 in the width direction X. The colored region A1 is a region where the colored tissue 33 is disposed, and is a region where the color of the colored tissue 33 (blue in the present embodiment) is visible through when viewed from the skin-facing side Z1 of the top sheet 21. The non-colored region A2 is a region where the colored tissue 33 is not disposed, and is a region where the white color (uncolored) of the white tissue 32 is visible through when viewed from the skin-facing side Z1 of the top sheet 21. That is, in the present embodiment, the colored tissue 33 functions as a member constituting the colored region A1, and the white tissue 32 mainly functions as a member constituting the non-colored region A2 (white region).

The pair of side sheets 24 is positioned at the end portions of the inner body 20 in the width direction X. The pair of side sheets 24 forms a pair of leakage-proof gathers 25 for preventing excreta such as urine excreted by the wearer from leaking in the width direction X (lateral leakage). The pair of leakage-proof gathers 25 is provided on both sides of the absorbent body 23 in the width direction X, with both end portions (fixed regions FA) in the longitudinal direction Y being fixed, and is configured to be able to stand upright by the portion between the fixed regions FA contracting in the longitudinal direction Y. As an example, the leakage-proof gather 25 has a structure in which a plurality (for example, four) of stretchable members 26 extending in the longitudinal direction Y are disposed inside the side sheet 24 folded back in a bag shape. Note that in FIG. 3, only one stretchable member 26 out of the four stretchable members 26 included in each leakage-proof gather 25 shown in FIG. 5 is illustrated. By the stretchable member 26 contracting in the longitudinal direction Y in this manner, the portion of the side sheet 24 disposed between the fixed regions FA functions as the leakage-proof gather 25. The stretchable member 26 is formed of an elastic material such as rubber thread or flat rubber, for example.

### (Operation and Effect of Absorbent Article 1)

In the absorbent article 1 described above, as shown in FIG. 3, the outer edge portion (outer end portion 33a of the colored tissue 33) of each of the pair of colored regions A1 in the width direction X is disposed so as to overlap with the edge 31c of the absorbent core 31 in the width direction X. Furthermore, the quantity X1 of sheet materials laminated on the pair of colored regions A1 (colored tissues 33) on the side (skin-facing side Z1) opposite to the side (non-skin-facing side Z2) on which the absorbent core 31 is disposed with respect to the pair of colored regions A1 (colored tissues 33) is equal to or less than the quantity X2 of sheet materials laminated on the non-colored region A2 (white tissue 32) on the side (skin-facing side Z1) opposite to the side (non-skin-facing side Z2) on which the absorbent core 31 is disposed with respect to the non-colored region A2 (white tissue 32) (that is, "X1 ≤ X2"). In the present embodiment, as shown in FIG. 5, since the only sheet material disposed on the colored region A1 (colored tissue 33) is the top sheet 21, "X1 = 1 top sheet 21". Similarly, since the only sheet material disposed on the non-colored region A2 (white tissue 32) is the top sheet 21, "X2 = 1 top sheet 21". Therefore, "X1 = X2" holds.

As described above, in the absorbent article 1, the pair of colored regions A1 having outer edge portions overlapping with the edges 31c of the absorbent core 31 in the width direction X is provided, and the quantity X1 of sheet materials laminated on the pair of colored regions A1 is equal to or less than the quantity X2 of sheet materials laminated on the non-colored region A2 (X1 = X2 in the present embodiment). That is, the absorbent article is configured such that the visibility of the pair of colored regions A1 is not impaired more than necessary by not disposing a greater quantity of sheet materials on the pair of colored regions A1 than on the non-colored region A2. This allows the user to appropriately visually recognize the pair of colored regions A1, and allows the user to easily grasp the position of the edges 31c of the absorbent core 31 in the width direction X. As a result, it is possible to give the user a sense of expectation that leakage of excreta will be effectively prevented by the absorbent core 31 disposed up to the positions overlapping with the outer edge portions (outer end portions 33a of the colored tissues 33) of the pair of colored regions A1. Furthermore, after use of the absorbent article 1 or the like, when it is confirmed that lateral leakage of excreta has not occurred, a sense of security with respect to the absorbent article 1 (in other words, a sense of trust in the absorbent article 1 based on the actual feeling that the effect that was expected in advance as described above was actually obtained) can be suitably fostered. Therefore, according to the absorbent article 1, the user's sense of security can be effectively improved.

In addition, the absorbent article is configured such that the quantity of sheet materials laminated in the region along the edge (outer end portion 33a of the colored tissue 33) of the colored region A1 in the width direction X is not greater than in other regions within the colored region A1. That is, the entire colored region A1 has the same visibility, and the region along the edge (outer end portion 33a) of the colored region A1 that the user particularly wants to visually recognize is configured so as not to have its visibility impaired more than other regions within the colored region A1. This allows the user to easily and visually grasp the position of the edge 31c of the absorbent core 31 through the colored region A1.

The pair of colored regions A1 and the non-colored region A2 are disposed on the skin-facing side Z1 of the wearer relative to the absorbent core 31. According to the above configuration, when the absorbent article 1 is put on the wearer (that is, in a scene where the user visually recognizes the skin-facing side Z1 of the absorbent article 1), by allowing the user to visually grasp the region where the absorbent core 31 is disposed (the position of the edge 31c in the width direction X) through the pair of colored regions A1, a sense of security with respect to the absorbent article 1 can be suitably given to the user.

As shown in (B) of FIG. 5, in a state where the leakage-proof gathers 25 are folded toward the inner side in the width direction X, the inner edge portion of the colored region A1 in the width direction X (that is, the inner end portion 33c of the colored tissue 33) is positioned further inward in the width direction X than the top portions 25a of the leakage-proof gathers 25. According to the above configuration, even in a state where the leakage-proof gathers 25 are laid down to the maximum extent toward the inner side in the width direction X, the colored region A1 is not completely hidden by the leakage-proof gathers 25, and thus the visibility of the colored region A1 can be ensured.

As shown in (B) of FIG. 5, in a state where the leakage-proof gathers 25 are folded toward the inner side in the width direction X, at the central position of the absorbent body 23 in the longitudinal direction Y (on the center line CL2 in FIG. 3), the edges 31c of the absorbent core 31 in the width direction X are positioned further outward in the width direction X than the top portions 25a of the leakage-proof gathers 25. According to the above configuration, by forming the absorbent core 31 in a wide shape (for example, a shape with no general constriction, or a shape in which the width of the constricted portion is larger than the general size even if there is a constricted portion) such that the absorbent core 31 has the above-described positional relationship with respect to the leakage-proof gathers 25, the effect of preventing lateral leakage of excreta by the absorbent core 31 can be enhanced. Furthermore, since the fact that the absorbent core 31 is wide enough that the positions of the edges 31c have the above-described positional relationship with respect to the leakage-proof gathers 25 is visually emphasized by the colored region A1, a sense of security with respect to the absorbent article 1 can be suitably given to the user. Furthermore, according to the configuration in which the absorbent core 31 is formed in a wide shape in this manner and the colored regions A1 are provided at positions along the edges 31c of the absorbent core 31, even when an absorbent pad such as a urine-absorbing pad is disposed on the inner body 20 (top sheet 21) of the absorbent article 1, it is possible to make the colored regions A1 protrude from both sides of the absorbent pad in the width direction X, allowing the user to visually recognize that the absorbent core 31 is wide. As a result, even when the absorbent article 1 and the absorbent pad are used together, a sense of security with respect to the absorbent article 1 can be suitably given to the user.

Furthermore, the absorbent core 31 is formed with a substantially uniform width over the entire region in the longitudinal direction Y. In the present embodiment, the absorbent core 31 is formed in a rectangular shape with a uniform width when viewed from the thickness direction Z. According to the above configuration, since the absorbent core 31 does not have a constricted shape and is formed with a wide width over the entire region in the longitudinal direction Y, the lateral leakage prevention effect and the sense of security given to the user can be improved.

As shown in FIGS. 3 and 5, the portion of the absorbent core 31 included in the crotch region 1c is provided with a pair of recessed portions 311 on both sides of the center line CL1, each extending in the longitudinal direction Y and having a thickness of the absorbent core 31 in the thickness direction Z smaller than that of the surrounding area. As an example, the recessed portions 311 can be formed by laminating the materials constituting the absorbent core 31 (SAP, pulp, and the like) only in the region excluding a part of the region on the back sheet 22 (the region where the recessed portions 311 are to be formed). In this case, the recessed portions 311 are portions where the materials constituting the absorbent core 31 are not present and the thickness of the absorbent core 31 is zero (thickness is 0). According to the above configuration, since it is possible to deform the absorbent core 31 using the pair of recessed portions 311 as starting points, it is possible to improve the lateral leakage prevention effect by the wide absorbent core 31 while reducing the discomfort felt by the wearer when wearing the absorbent article 1, which may be caused by making the absorbent core 31 wide.

In the present embodiment, each of the pair of recessed portions 311 has a ventral curved portion 311a that curves toward the outer side in the width direction X as it extends from the central position of the recessed portion 311 in the longitudinal direction Y toward the ventral side, and a dorsal curved portion 311b that curves toward the outer side in the width direction X as it extends from the central position of the recessed portion 311 toward the dorsal side. That is, each recessed portion 311 has an arc shape in which the central position in the longitudinal direction Y is positioned at the innermost side in the width direction X and both end positions in the longitudinal direction Y are positioned at the outermost side in the width direction X. According to the above configuration, the pair of recessed portions 311 each having the ventral curved portion 311a and the dorsal curved portion 311b can improve the fit around the legs of the wearer, and as a result, the discomfort felt by the wearer during wear can be effectively reduced.

As shown in FIG. 3, in the present embodiment, the entirety of each of the pair of recessed portions 311 overlaps with the colored region A1 in the thickness direction Z. As an example, the central position of the recessed portion 311 in the longitudinal direction Y (the portion positioned at the innermost side in the width direction X) is disposed so as to be in contact with the inner end portion (inner end portion 33c of the colored tissue 33) of the colored region A1 when viewed from the thickness direction Z. According to the above configuration, by overlapping the recessed portions 311, which serve as the deformation starting points of the absorbent article 1, with the colored region A1, the colored region A1 can be suitably deformed. This makes it easier for the user to visually recognize how the colored region A1 (that is, the absorbent core 31) appropriately deforms in accordance with the movement around the legs. As a result, a sense of security with respect to the absorbent article 1 can be suitably given to the user.

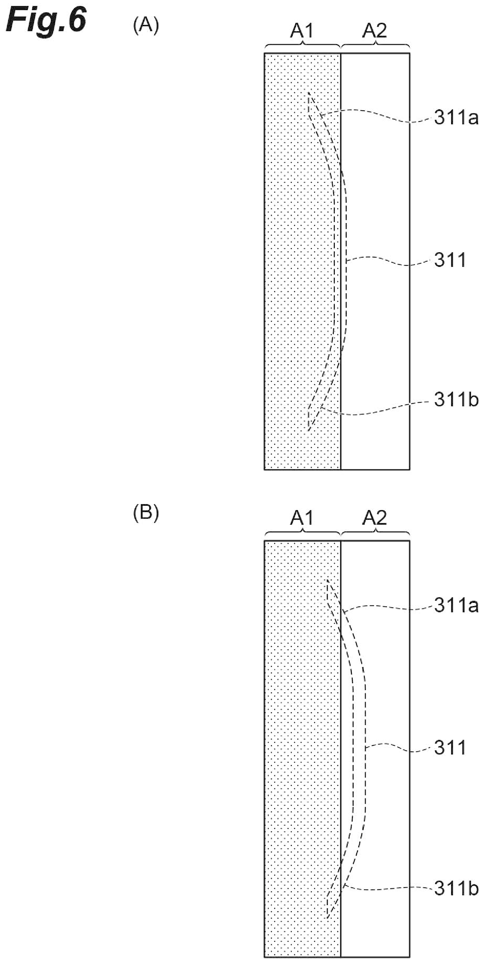

Note that, as shown in (A) of FIG. 6, each of the pair of recessed portions 311 may be disposed so as to overlap with the colored region A1 in the thickness direction Z over more than half of its area. In this example, the portion of the recessed portion 311 positioned at the innermost side in the width direction X is positioned further inward than the inner end portion 33c of the colored region A1 (colored tissue 33) when viewed from the thickness direction Z, so that more than half of the total area of the recessed portion 311 overlaps with the colored region A1. Alternatively, as shown in (B) of FIG. 6, at least a part of each of the pair of recessed portions 311 may be disposed so as to overlap with the colored region A1 in the thickness direction Z. In this example, only the respective tip portions of the ventral curved portion 311a and the dorsal curved portion 311b of the recessed portion 311 overlap with the colored region A1. Even in the form shown in (A) or (B) of FIG. 6, the above-described effect of making it easier for the user to visually recognize how the colored region A1 (that is, the absorbent core 31) appropriately deforms in accordance with the movement around the legs can be obtained. However, from the viewpoint of obtaining the above effect more suitably, the form of (B) of FIG. 6 is more preferable than the form of (C) of FIG. 6, and the form of the above embodiment (FIG. 3) is even more preferable than the form of (B) of FIG. 6.

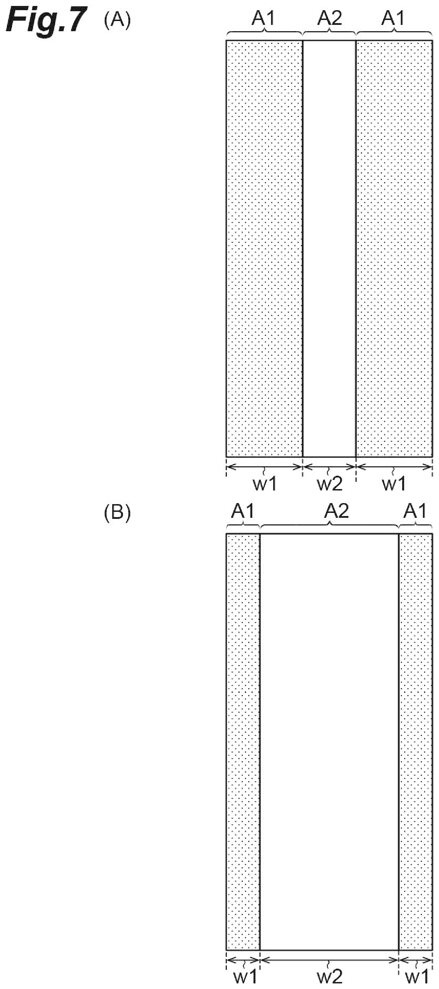

At the central position of the absorbent body 23 in the longitudinal direction Y (on the center line CL2), the sum of the widths w1 of the pair of colored regions A1 in the width direction X (w1 × 2) may be equal to or greater than the width (w2) of the non-colored region A2 in the width direction X. That is, the width w1 of the pair of colored regions A1 may be set such that "w1 × 2 ≥ w2" holds. For example, as shown in (A) of FIG. 7, the configuration may be such that the sum of the widths w1 of the colored regions A1 is greater than the width w2 of the non-colored region A2. According to the above configuration, by making the widths of the pair of colored regions A1 sufficiently large relative to the non-colored region A2, the visibility of the pair of colored regions A1 can be effectively enhanced.

Alternatively, at the central position of the absorbent body 23 in the longitudinal direction Y (on the center line CL2), the sum of the widths w1 of the pair of colored regions A1 in the width direction X may be equal to or less than the width w2 of the non-colored region A2 in the width direction X. That is, the width w1 of the pair of colored regions A1 may be set such that "w1 × 2 ≤ w2" holds. For example, as shown in (B) of FIG. 7, the configuration may be such that the sum of the widths w1 of the colored regions A1 is smaller than the width w2 of the non-colored region A2. According to the above configuration, by keeping the widths of the pair of colored regions A1 to a certain level or less relative to the non-colored region A2, the amount of coloring material required to form the pair of colored regions A1 can be reduced. In addition, by setting the colored regions A1 only at positions relatively close to the portion along the edge 31c of the absorbent core 31, it becomes possible to allow the user to more accurately grasp the position of the edge 31c of the absorbent core 31 through the colored regions A1.

As shown in FIG. 3, in the present embodiment, the width w1 of each colored region A1 is set such that the sum of the widths w1 of the pair of colored regions A1 (w1 × 2) is equal to the width w2 of the non-colored region A2. According to this configuration, both the advantage of the case of "w1 × 2 ≥ w2" and the advantage of the case of "w1 × 2 ≤ w2" described above can be obtained in a well-balanced manner.

It is preferable that each of the pair of colored regions A1 is disposed over at least the entire region of the crotch region 1c in the longitudinal direction Y. In the present embodiment, the ventral end portion 31a and the dorsal end portion 31b of the absorbent core 31 reach the ventral region 1a and the dorsal region 1b, respectively, and both end portions in the longitudinal direction Y of each colored region A1 (both edges 33b of the colored tissue 33) overlap with the ventral end portion 31a and the dorsal end portion 31b of the absorbent core 31, and thus the above arrangement condition is satisfied. According to the above configuration, by disposing the colored regions A1, which serve as markers for the edge positions of the absorbent core 31, over the entire crotch region 1c, which is the portion where lateral leakage of excreta is most likely to occur (that is, the portion where the user is most likely to worry about the occurrence of lateral leakage), the user's sense of security can be suitably fostered.

As shown in FIG. 3, it is preferable that each of the pair of colored regions A1 is disposed so as to overlap with the regions on both sides of the central region R1 in the longitudinal direction Y (the region R2 on the ventral side and the region R3 on the dorsal side) when the absorbent article 1 is divided into three equal parts in the longitudinal direction Y. In the present embodiment, the ventral region 1a is included in the region R2, the dorsal region 1b is included in the region R3, and each colored region A1 extends from the ventral region 1a to the dorsal region 1b, and thus the above arrangement condition is satisfied. According to the above configuration, by making the length of each colored region A1 in the longitudinal direction Y sufficiently long, the visibility of the colored region A1 can be improved and the user's sense of security can be suitably fostered. Note that from the viewpoint of further enhancing the above effect, it is more preferable that each of the pair of colored regions A1 is disposed so as to overlap with the regions on both sides in the longitudinal direction Y (the regions on both sides excluding the two central regions) when the absorbent article 1 is divided into four equal parts in the longitudinal direction Y.

The edges in the longitudinal direction Y of each of the pair of colored regions A1 (edges 33b of the colored tissue 33) overlap with the edges of the absorbent core 31 in the longitudinal direction Y (the ventral end portion 31a and the dorsal end portion 31b). According to the above configuration, by making the length of each colored region A1 in the longitudinal direction Y sufficiently long to be the same length as the absorbent core 31, the visibility of the colored region A1 can be improved and the user's sense of security can be suitably fostered.

As shown in FIGS. 3 and 5, the portion of the absorbent core 31 included in the crotch region 1c is provided with a groove portion 312 extending in the longitudinal direction Y along the center line CL1 and having a thickness of the absorbent core 31 in the thickness direction Z smaller than that of the surrounding area. As an example, the groove portion 312 can be formed, similarly to the pair of recessed portions 311, by laminating the materials constituting the absorbent core 31 (SAP, pulp, and the like) only in the region excluding a part of the region on the back sheet 22 (the region where the groove portion 312 is to be formed). In this case, the groove portion 312 is formed of a portion where the materials constituting the absorbent core 31 are not present and the thickness of the absorbent core 31 is zero (thickness is 0). In the present embodiment, both the pair of recessed portions 311 and the groove portion 312 are formed by laminating the materials constituting the absorbent core 31 only in the region excluding the regions where the pair of recessed portions 311 and the groove portion 312 are to be formed. In addition, the groove portion 312 is disposed between the pair of recessed portions 311 at a position facing the urethral opening of the wearer. As an example, the groove portion 312 is formed in a rectangular shape that is elongated in the longitudinal direction Y when viewed from the thickness direction Z.

According to the above configuration, urine excreted by the wearer (urine that has passed through the top sheet 21) can be suitably diffused along the groove portion 312 in the longitudinal direction Y of the absorbent core 31, and thus the absorption efficiency of urine in the absorbent core 31 can be improved. In addition, since the absorbent core 31 can be deformed along the groove portion 312, the adhesion of the absorbent core 31 to the urethral opening of the wearer can be improved. Furthermore, by forming the absorbent core 31 in a wide shape and providing the groove portion 312 as in the present embodiment, it is possible to improve the adhesion of the absorbent core 31 to the urethral opening of the wearer while sufficiently increasing the area of the absorbent core 31 existing on the outer side of the adhesion portion (the portion facing the urethral opening) in the width direction X. This can effectively reduce the risk of urine leakage and give the user an even greater sense of security with respect to the absorbent article 1.

As shown in FIGS. 3 and 5, the inner body 20 has a stretchable member 27 for stretching and contracting, in the longitudinal direction Y, at least a part of the portion of the absorbent core 31 included in the crotch region 1c and extending along the center line CL1. The stretchable member 27 is formed of an elastic material such as rubber thread or flat rubber, for example, similarly to the stretchable member 26. In the present embodiment, as an example, the above-described top sheet 21 is formed in a bag shape so as to cover the side surfaces of the absorbent core 31 and the surface on the non-skin-facing side Z2 of the back sheet 22. The stretchable member 27 is fixed and disposed between the back sheet 22 and the top sheet 21 on the non-skin-facing side Z2 of the back sheet 22. This can suppress the sagging of the adhesion portion of the absorbent core 31 (the portion that deforms along the groove portion 312 so as to adhere to the urethral opening) (that is, movement in the direction away from the urethral opening), and can effectively improve the adhesion of the absorbent core 31 to the urethral opening of the wearer.

Furthermore, in the present embodiment, as an example, the stretchable member 27 is disposed at a position corresponding to the groove portion 312 (in the present embodiment, a position along the edge of the groove portion 312) when viewed from the thickness direction Z. More specifically, a pair of stretchable members 27 each extending in the longitudinal direction Y are disposed along both side edges of the groove portion 312 in the width direction X. This can more effectively suppress the sagging of the adhesion portion of the absorbent core 31 (the portion that deforms along the groove portion 312 so as to adhere to the urethral opening) (that is, movement in the direction away from the urethral opening), and can more effectively improve the adhesion of the absorbent core 31 to the urethral opening of the wearer.

### [Modification Example]

Although the embodiments of the present disclosure have been described above, the present disclosure is not limited to the above embodiments. The material and shape of each configuration are not limited to the above-described material and shape, and various materials and shapes can be adopted. In addition, a part of the configuration of the above-described absorbent article 1 may be modified into other configurations or omitted.

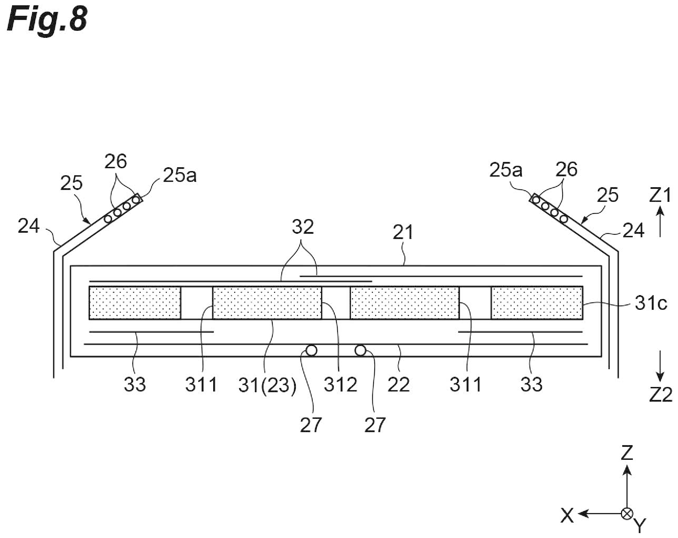

In the above embodiment, the pair of colored regions A1 and the non-colored region A2 were disposed on the skin-facing side Z1 relative to the absorbent core 31. In contrast, for example, as shown in FIG. 8, the pair of colored regions A1 and the non-colored region A2 may be disposed on the non-skin-facing side Z2 relative to the absorbent core 31. In the example of FIG. 8, the colored tissue 33 constituting the colored region A1 is disposed between the absorbent core 31 and the back sheet 22. For this reason, while the quantity X1 of sheet materials laminated on the colored region A1 (colored tissue 33) on the non-skin-facing side Z2 is only the quantity of the back sheet 22 and the top sheet 21 (the portion covering the back sheet 22), the quantity X2 of sheet materials laminated on the non-colored region A2 (white tissue 32) on the non-skin-facing side Z2 is the quantity obtained by adding the absorbent core 31 to the back sheet 22 and the top sheet 21 (the portion covering the back sheet 22), and thus "X1 < X2" holds. Note that even when the white color of the non-colored region A2 is considered to be constituted by the absorbent core 31, "X1 = X2" holds, and at least "X1 > X2" does not hold. According to the above configuration, when the absorbent article 1 is taken out from a package (for example, a package accommodating a plurality of absorbent articles 1) or after the absorbent article 1 is put on the wearer (that is, in a scene where the user visually recognizes the non-skin-facing side Z2 of the absorbent article 1), by allowing the user to visually grasp the region where the absorbent core 31 is disposed (the position of the edge 31c in the width direction X) through the pair of colored regions A1, a sense of security with respect to the absorbent article 1 can be suitably given to the user.

Note that the above embodiment and the configuration of FIG. 8 may be combined. That is, as in the above embodiment, the colored tissue 33 may be disposed on the skin-facing side Z1 relative to the absorbent core 31, and as in the example of FIG. 8, the colored tissue 33 may also be disposed on the non-skin-facing side Z2 relative to the absorbent core 31. In this case, from both the skin-facing side Z1 and the non-skin-facing side Z2, it becomes possible to allow the user to visually grasp the position of the edge 31c of the absorbent core 31 through the colored region A1 constituted by the colored tissue 33, and a sense of security with respect to the absorbent article 1 can be even more suitably given to the user.

In the above embodiment, the shape, width (length in the width direction X), and length (length in the longitudinal direction Y) of one colored region A1 are the same as the shape, width, and length of the other colored region A1, but at least one of the shape, width, and length of one colored region A1 may be different from at least one of the shape, width, and length of the other colored region A1.

In the above embodiment, the colored region A1 is realized by disposing the colored tissue 33 as a separate member from the absorbent core 31 and the white tissue 32, but the colored region A1 may be formed by printing (for example, color printing on a part of the white tissue 32). When the colored region A1 is formed by printing, the number of members required to form the colored region A1 can be reduced.

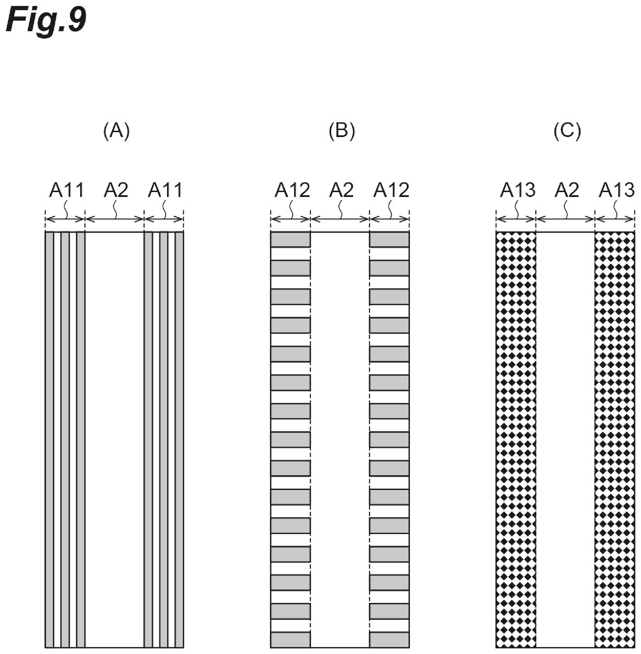

In addition, when printing is used, it becomes easy to form colored regions A11, A12, and A13 having predetermined patterns as shown in FIG. 9. The colored region A11 shown in (A) of FIG. 9 is formed by arranging a plurality of vertically striped colored portions. The colored region A12 shown in (B) of FIG. 9 is formed by arranging a plurality of horizontally striped colored portions. The colored region A13 shown in (C) of FIG. 9 is formed by regularly arranging a plurality of dot-shaped colored portions. Note that when a colored region is constituted by a plurality of scattered colored portions in this manner, a region including the plurality of colored portions (that is, one region that can be perceived as a single region in which the plurality of colored portions are arranged in a predetermined pattern) can be defined as a region (colored regions A11 to A13) corresponding to the colored region A1 described in the above embodiment.

In addition, in the above embodiment, the absorbent article 1 is a pants-type diaper, but the absorbent article 1 may be a tape-type diaper. In addition, the absorbent article 1 may be an absorbent pad having the same or similar configuration as the inner body 20 of the above embodiment and not including a configuration corresponding to the outer body 10 of the above embodiment. Note that when the absorbent article 1 is configured as a tape-type diaper or an absorbent pad, the "unfolded state" of the absorbent article 1 described in the above embodiment can be rephrased as a state in which the absorbent article 1 is extended to a state where no wrinkles are formed.

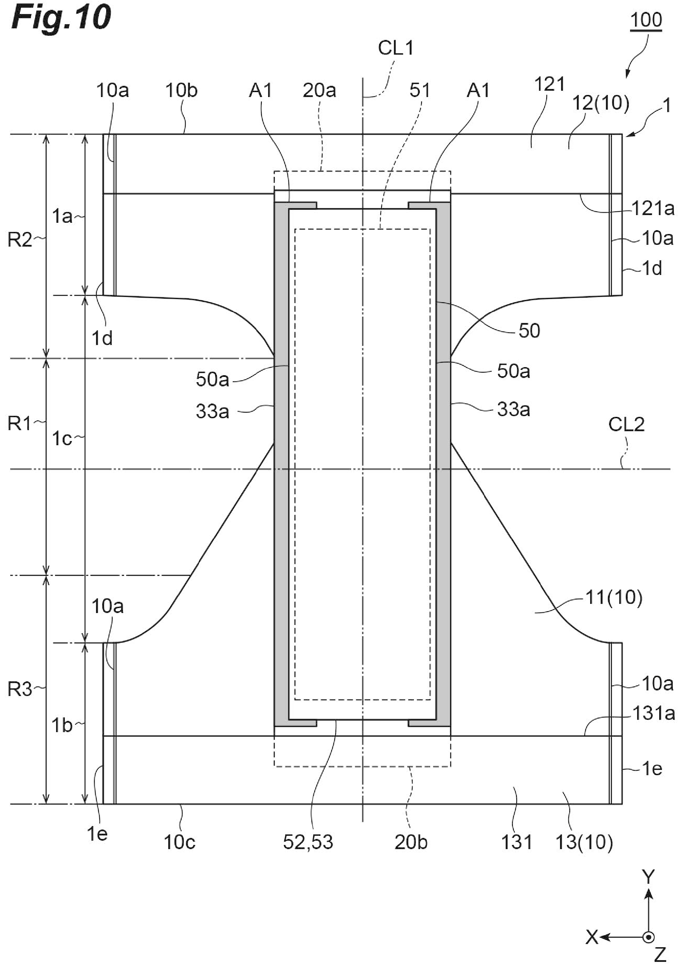

As shown in FIG. 10, the above-described absorbent article 1 may be combined with an absorbent pad 50 to constitute an absorbent article set 100. The absorbent pad 50 has an absorbent core 51, a top sheet 52 covering the skin-facing side Z1 of the absorbent core 51, and a back sheet 53 covering the non-skin-facing side Z2 of the absorbent core 51. The absorbent core 51 can be formed of, for example, the same materials as the absorbent core 31 (for example, SAP, pulp, and the like). As an example, the absorbent pad 50 has a rectangular shape when viewed from the thickness direction Z. However, the shape of the absorbent pad 50 is not limited to the above. For example, the absorbent pad 50 may be formed in an hourglass shape, or may be formed in a shape asymmetric with respect to a central axis parallel to the longitudinal direction Y.

In the absorbent article set 100, in a state where the absorbent pad 50 is attached to the skin-facing side Z1 of the inner body 20 of the absorbent article 1 such that the center line CL1 passes through the center of the absorbent pad 50 in the width direction X, at the central position of the absorbent body 23 in the longitudinal direction Y (that is, on the center line CL2), the outer edge portions (outer end portions 33a of the colored tissues 33) of the pair of colored regions A1 in the width direction X are positioned further outward than the outer end portions 50a of the absorbent pad 50 in the width direction X. As an example, a joining member for joining the absorbent pad 50 to the inner body 20 (top sheet 21) may be provided on the non-skin-facing side Z2 of the back sheet 53 of the absorbent pad 50. In this case, by disposing the absorbent pad 50 with respect to the inner body 20 such that the center line CL1 passes through the center of the absorbent pad 50 in the width direction X as described above, and pressing the back sheet 53 of the absorbent pad 50 against the top sheet 21 of the inner body 20, the absorbent pad 50 can be attached to the inner body 20.

In the absorbent article set 100 of the above aspect, even in a state where the absorbent pad 50 is attached to the inner body 20 of the absorbent article 1 for use in combination with the absorbent article 1, the user can visually recognize the outer edge portions (outer end portions 33a) of the pair of colored regions A1 at the central position of the absorbent body 23 in the longitudinal direction Y (on the center line CL2). This allows the user to visually grasp that the absorbent article 1 has an absorbent core 31 that is even wider than the absorbent pad 50 in the region where lateral leakage is of concern (that is, the central position of the absorbent body 23 in the longitudinal direction Y). As a result, the user's sense of security can be effectively improved.

Note that the absorbent article constituting the absorbent article set 100 does not need to have all the configurations of the absorbent article 1 of the above embodiment, and it is sufficient that at least the pair of colored regions A1 (the members constituting the colored regions A1) is disposed on the skin-facing side Z1 relative to the absorbent core 31 and has the above-described wide shape on the center line CL2. In addition, the absorbent article 1 may be a tape-type disposable diaper. Even in that case, the same effects as those described above for the case where the absorbent article 1 is a pants-type disposable diaper can be obtained. In addition, the absorbent article 1 and the absorbent pad 50 may constitute a group of articles having the same brand name (a notation given to products of the same concept). That is, the absorbent article set 100 having the above-described configuration may be realized by combining the absorbent article 1 and the absorbent pad 50 that correspond to each other under the same brand name (and size). In this case, the user can easily obtain the absorbent article 1 and the absorbent pad 50 constituting the absorbent article set 100 using the same brand name as a guide (that is, realize the above-described absorbent article set 100 to obtain a sense of security). For example, the common brand name shared by the absorbent article 1 and the absorbent pad 50 (for example, "Lifree (registered trademark)") may be displayed on the absorbent article 1 itself (for example, the non-skin-facing side Z2 of the outer body 10) or on a package accommodating one or more absorbent articles 1. Similarly, the above-described common brand name may be displayed on the absorbent pad 50 itself or on a package accommodating one or more absorbent pads 50.

### Reference Signs List

1: Absorbent article
1a: Ventral region
1b: Dorsal region
1c: Crotch region
21: Top sheet
22: Back sheet
23: Absorbent body
25: Leakage-proof gather
25a: Top portion
27: Stretchable member
31: Absorbent core
31c: Edge
33b: Edge
311: Recessed portion
50: Absorbent pad
50a: Outer end portion
100: Absorbent article set
311a: Ventral curved portion
311b: Dorsal curved portion
312: Groove portion
A1, A11, A12, A13: Colored region
A2: Non-colored region

## Claims

1. An absorbent article in which a ventral region positioned on a ventral side of a wearer in a worn state, a dorsal region positioned on a dorsal side of the wearer in the worn state, and a crotch region positioned between the ventral region and the dorsal region are provided along a longitudinal direction, the absorbent article comprising:
a liquid-permeable top sheet positioned on a skin-facing side of the wearer in the worn state;
a liquid-impermeable back sheet positioned on a non-skin-facing side of the wearer in the worn state; and
an absorbent body disposed between the top sheet and the back sheet and including an absorbent core that absorbs excreta of the wearer,
wherein the absorbent body has:
a pair of colored regions disposed on both sides of a center line parallel to the longitudinal direction and passing through a center of the absorbent body in a width direction perpendicular to the longitudinal direction so as to overlap with the absorbent core in a thickness direction perpendicular to the longitudinal direction and the width direction; and
a non-colored region disposed between the pair of colored regions in the width direction on the same side as the side on which the pair of colored regions is provided with respect to the absorbent core,
wherein an outer edge portion of each of the pair of colored regions in the width direction is disposed so as to overlap with an edge of the absorbent core in the width direction, and
a quantity of sheet materials laminated on the pair of colored regions on a side opposite to the side on which the absorbent core is disposed with respect to the pair of colored regions is equal to or less than a quantity of sheet materials laminated on the non-colored region on a side opposite to the side on which the absorbent core is disposed with respect to the non-colored region.

2. The absorbent article according to claim 1, wherein the pair of colored regions and the non-colored region are disposed on the skin-facing side of the wearer relative to the absorbent core.

3. The absorbent article according to claim 1, wherein the pair of colored regions and the non-colored region are disposed on the non-skin-facing side of the wearer relative to the absorbent core.

4. The absorbent article according to claim 1, further comprising a pair of leakage-proof gathers provided on both sides of the absorbent body in the width direction, both end portions of the leakage-proof gathers in the longitudinal direction being fixed and a portion between both end portions being configured to be able to stand upright by contracting in the longitudinal direction,
wherein in a state where the leakage-proof gathers are folded toward an inner side in the width direction, an inner edge portion of each of the pair of colored regions in the width direction is positioned further inward in the width direction than top portions of the leakage-proof gathers.

5. The absorbent article according to claim 1, further comprising a pair of leakage-proof gathers provided on both sides of the absorbent body in the width direction, both end portions of the leakage-proof gathers in the longitudinal direction being fixed and a portion between both end portions being configured to be able to stand upright by contracting in the longitudinal direction,
wherein in a state where the leakage-proof gathers are folded toward an inner side in the width direction, at a central position of the absorbent body in the longitudinal direction, edges of the absorbent core in the width direction are positioned further outward in the width direction than top portions of the leakage-proof gathers.

6. The absorbent article according to claim 5, wherein the absorbent core is formed with a substantially uniform width over an entire region in the longitudinal direction.

7. The absorbent article according to claim 5 or 6, wherein a portion of the absorbent core included in the crotch region is provided with a pair of recessed portions on both sides of the center line, each of the recessed portions extending in the longitudinal direction and having a thickness of the absorbent core in the thickness direction smaller than that of a surrounding area.

8. The absorbent article according to claim 7, wherein each of the pair of recessed portions has a ventral curved portion that curves toward an outer side in the width direction as it extends from the central position of the recessed portion in the longitudinal direction toward the ventral side, and a dorsal curved portion that curves toward the outer side in the width direction as it extends from the central position of the recessed portion toward the dorsal side.

9. The absorbent article according to claim 7, wherein at least a part of each of the pair of recessed portions overlaps with a corresponding one of the pair of colored regions in the thickness direction.

10. The absorbent article according to claim 7, wherein each of the pair of recessed portions overlaps with a corresponding one of the pair of colored regions in the thickness direction over more than half of its area.

11. The absorbent article according to claim 7, wherein an entirety of each of the pair of recessed portions overlaps with a corresponding one of the pair of colored regions in the thickness direction.

12. The absorbent article according to claim 1, wherein at a central position of the absorbent body in the longitudinal direction, a sum of widths of the pair of colored regions in the width direction is equal to or greater than a width of the non-colored region in the width direction.

13. The absorbent article according to claim 1, wherein at a central position of the absorbent body in the longitudinal direction, a sum of widths of the pair of colored regions in the width direction is equal to or less than a width of the non-colored region in the width direction.

14. The absorbent article according to claim 1, wherein each of the pair of colored regions is disposed over at least an entire region of the crotch region in the longitudinal direction.

15. The absorbent article according to claim 1, wherein each of the pair of colored regions is disposed so as to overlap with regions on both sides of a central region in the longitudinal direction when the absorbent article is divided into three equal parts in the longitudinal direction.

16. The absorbent article according to claim 1, wherein edges of each of the pair of colored regions in the longitudinal direction overlap with edges of the absorbent core in the longitudinal direction.

17. The absorbent article according to claim 1, wherein a portion of the absorbent core included in the crotch region is provided with a groove portion extending in the longitudinal direction along the center line and having a thickness of the absorbent core in the thickness direction smaller than that of a surrounding area.

18. The absorbent article according to claim 1, further comprising a stretchable member for stretching and contracting, in the longitudinal direction, at least a part of the portion of the absorbent core included in the crotch region and extending along the center line.

19. An absorbent article set including the absorbent article according to claim 2 and an absorbent pad used by being attached to the absorbent article,
wherein the absorbent article is a pants-type or tape-type disposable diaper, and
in a state where the absorbent pad is attached to the skin-facing side of the absorbent article such that the center line passes through the center of the absorbent pad in the width direction, at a central position of the absorbent body in the longitudinal direction, outer edge portions of the pair of colored regions in the width direction are positioned further outward than outer end portions of the absorbent pad in the width direction.
